# EUROPEAN PATENT APPLICATION

(11) **EP 4 582 785 A1**
(43) Date of publication of application: **09.07.2025**
(21) Application number: 23868232.2
(22) Date of filing: 21.09.2023
(51) Int. Cl.: G01N 1/00, G01N 1/28, C12M 1/26

(54) **COLLECTING KIT JIG**

(30) Priority: 21.09.2022 JP 2022150007
(71) Applicant: Terumo Kabushiki Kaisha, Tokyo 151-0072 (JP)
(72) Inventor: OKABE, Hiroshi, Fujinomiya-shi, Shizuoka 418-0004 (JP)
(74) Representative: TBK
(86) International application number: PCT/JP2023/034180
(87) International publication number: WO 2024/063111

(57) **Abstract**

A collection kit jig (100) includes a kit holding unit (102), a first adapter supporting unit (104) that fixes a first adapter (21a), a second adapter supporting unit (106) that fixes a second adapter (21b), a first bottle fixing unit (108) that fixes a first culture bottle (90), a second bottle fixing unit (110) that fixes a second culture bottle (92), and an elevation mechanism (112) that relatively moves the kit holding unit (102) in a vertical direction with respect to the first bottle fixing unit (108) and the second bottle fixing unit (110).

## Description

### Technical Field

The present invention relates to a collection kit jig used for connecting a collection kit to a culture bottle.

### Background Art

Blood products include a red blood cell product, a plasma product, a platelet product, and a whole blood product. As the blood products, products containing components required by patients are used for blood transfusion. The blood products are stored and transported in the state of being accommodated in medical bags. In order to ensure safety of the blood products, a small amount of sample may be collected to perform a culture test. In the culture test, the sample is collected in a culture bottle, and the culture bottle is placed in an environment suitable for bacterial growth to detect the presence or absence of a pathogen.

For example, a test for the platelet product is performed by the following procedure. A collection bag with a small volume is connected to a medical bag accommodating the platelet product (hereinafter, referred to as a platelet bag). Thereafter, a part of the platelet product in the platelet bag is transferred to the collection bag. Next, the collection bag is disconnected from the platelet bag.

Next, the collection bag is conveyed to a clean bench. In the clean bench, the platelet product in the collection bag is infused into a blood culture bottle. Prior to the infusion, a tube of the collection bag is connected to a tube extending from a sample collection tube. A prescribed amount of the platelet product is transferred from the collection bag to the sample collection tube with reference to scales of the sample collection tube.

Thereafter, an operator operates a valve of the sample collection tube while visually checking the scales of the sample collection tube to transfer a prescribed amount of the platelet product from the sample collection tube to the blood culture bottle. In the culture test, anaerobic culture and aerobic culture are performed. Therefore, the platelet product is dispensed into a culture bottle used for the anaerobic culture and a culture bottle used for the aerobic culture. Thereafter, the culture bottles are set in a culturing apparatus and subjected to the culture test for a predetermined period.

For example, US 8777921 discloses a collection kit for collecting a sample from a medical bag.

### Summary of Invention

As a kit for collecting a prescribed amount of sample from the blood product bag, a collection kit including an inlet tube, a collection bag, an outlet port, and an adapter is conceivable. When a large amount of a culture test of a blood product is performed, it is required to improve work efficiency of the culture test. For this reason, it is desirable that the adapter and the culture bottle can be easily connected.

An object of the present invention is to solve the above problem.

(1) One aspect of the present invention provides a collection kit jig used for connecting a collection kit to a culture bottle, the collection kit including an inlet tube to which a medical bag containing an object to be sampled is connected, a collection bag connected downstream of the inlet tube, having a first accommodation chamber and a second accommodation chamber, and having a connection flow path connecting the first accommodation chamber and the second accommodation chamber, a first outlet port connected to the first accommodation chamber, a second outlet port connected to the second accommodation chamber, a first adapter connected downstream of the first outlet port and connectable to a first culture bottle, and a second adapter connected downstream of the second outlet port and connectable to a second culture bottle, the collection kit jig including: a kit holding unit that holds the collection kit; a first adapter supporting unit that is provided in the kit holding unit and supports the first adapter; a second adapter supporting unit that is provided in the kit holding unit and supports the second adapter; a first bottle fixing unit that is disposed below the kit holding unit and fixes the first culture bottle; a second bottle fixing unit that is disposed below the kit holding unit and fixes the second culture bottle; and an elevation mechanism that moves the kit holding unit in a vertical direction relative to the first bottle fixing unit and the second bottle fixing unit.
(2) In the collection kit jig according to (1), the kit holding unit may include: a base portion fixed to the elevation mechanism; and a plurality of retaining pins that is insertable into retaining holes formed in the collection bag of the collection kit and that protrudes from the base portion.
(3) In the collection kit jig according to (2), the collection bag may have four corners arranged so as to surround the first accommodation chamber and the second accommodation chamber, and the plurality of retaining pins may include four retaining pins that retain the four corners of the collection bag.
(4) In the collection kit jig according to (1) or (3), the kit holding unit may include a base portion fixed to the elevation mechanism, the first adapter supporting unit may include a pair of first protrusions protruding from the base portion and separated from each other in a horizontal direction, and hold the first adapter between the pair of first protrusions, and the second adapter supporting unit may include a pair of second protrusions protruding from the base portion and separated from each other in the horizontal direction, and hold the second adapter between the pair of second protrusions.
(5) In the collection kit jig according to any one of (1) to (4), the collection bag may have a tubular portion forming an intermediate portion of the connection flow path, and the kit holding unit may include a clamp unit that closes the connection flow path when the tubular portion is inserted.
(6) In the collection kit jig according to (5), the clamp unit may have a clamp groove that has a V shape with an apex facing downward.
(7) In the collection kit jig according to any one of (1) to (6), the elevation mechanism may move the kit holding unit in the vertical direction.
(8) In the collection kit jig according to any one of (1) to (6), the elevation mechanism may move the first bottle fixing unit and the second bottle fixing unit in the vertical direction.

According to the present invention, the collection kit is held by the kit holding unit, the first adapter and the second adapter are fixed to the first adapter supporting unit and the second adapter supporting unit, respectively, the first culture bottle and the second culture bottle are fixed to the first bottle fixing unit and the second bottle fixing unit, respectively, and the kit holding unit holding the collection kit is lowered by the elevation mechanism, whereby the first adapter and the second adapter are connected to the first culture bottle and the second culture bottle, respectively. Therefore, the object to be sampled can be easily transferred from the collection kit to the two culture bottles.

### Brief Description of Drawings

Fig. 1 is a perspective view of a collection kit jig according to a first embodiment of the present invention.
Fig. 2 is an overall diagram of a collection kit.
Fig. 3 is a detailed diagram of an elevation mechanism in the collection kit jig.
Fig. 4 is a diagram of a state in which a sample is collected in a collection kit.
Fig. 5 is a diagram illustrating a state in which the collection kit is connected to a culture bottle.
Fig. 6 is an overall diagram of a collection kit according to anther configuration.
Fig. 7 is a perspective view of a collection kit jig according to a second embodiment of the present invention.
Fig. 8 is a perspective view of a collection kit jig according to a third embodiment of the present invention.
Fig. 9 is a perspective view of a collection kit jig according to a fourth embodiment of the present invention.

### Description of Embodiments

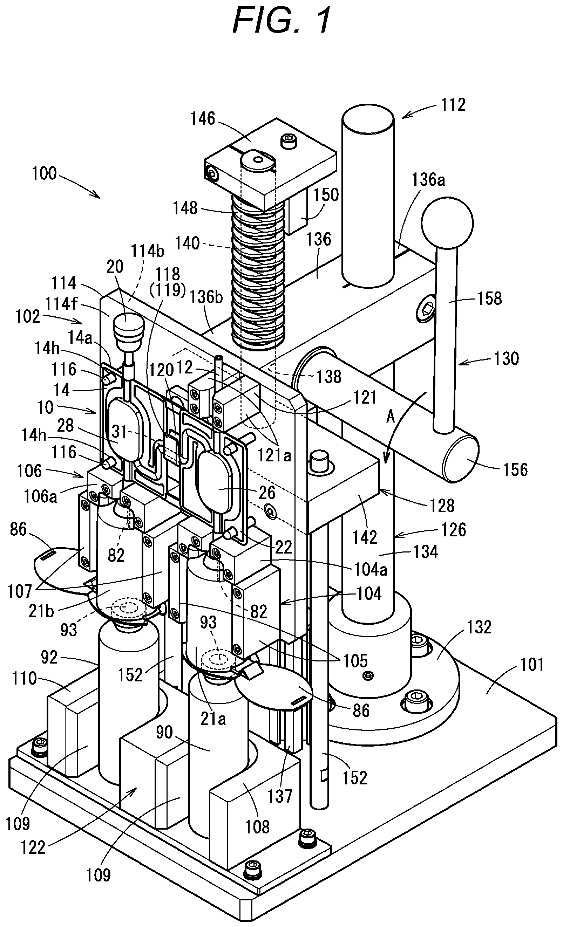

A collection kit jig 100 according to a first embodiment illustrated in Fig. 1 is used for connecting a collection kit 10 to a first culture bottle 90 and a second culture bottle 92. The collection kit 10 is used to transfer a blood product that is an object to be sampled to the culture bottles 90 and 92. The collection kit 10 is used for, for example, a culture test for confirming the safety of a blood product in a business place such as a blood center where blood products are manufactured. In the culture test, a test for anaerobic culture and a test for aerobic culture are performed. Therefore, in the culture test, two bottles are used, a culture bottle 90 (hereinafter referred to as "first culture bottle 90") for anaerobic culture and a culture bottle 92 (hereinafter referred to as "second culture bottle 92") for aerobic culture. The collection kit 10 is used to collect a liquid sample (platelet product) and dispense the sample into the two culture bottles 90 and 92 each in a prescribed amount.

In the following, the present embodiment will describe an example of using a platelet product as a blood product, but the blood product collected by the collection kit 10 may be red blood cell product, a plasma product, or a whole blood product.

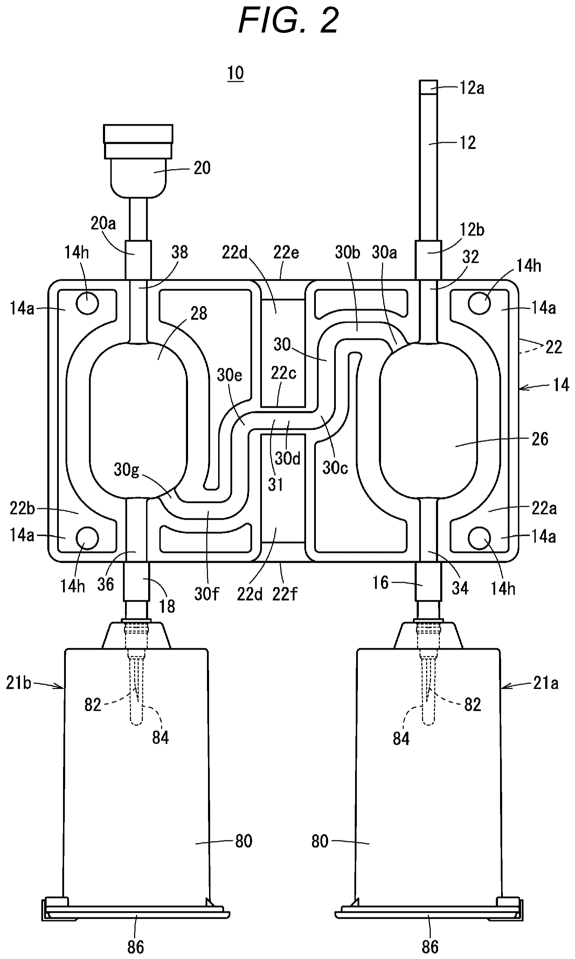

As illustrated in Fig. 2, the collection kit 10 includes an inlet tube 12, a collection bag 14, a first sealing member 16, a second sealing member 18, an exhaust valve 20, a first adapter 21a, and a second adapter 21b. In the collection kit 10, a direction in which the inlet tube 12 and the exhaust valve 20 are located is referred to as an upper side or upward direction, and a direction in which the first adapter 21a and the second adapter 21b are located is referred to as a lower side or downward direction based on an arrangement state during use. In addition, a direction perpendicular to the vertical direction and along a plane of the collection bag 14 is referred to as a width direction, and a direction perpendicular to the plane of the collection bag 14 is referred to as a thickness direction.

The inlet tube 12 is, for example, a translucent medical tube made of a thermoplastic resin such as a vinyl chloride resin. The inlet tube 12 can be connected to or separated from another medical tube without exposing the inside thereof to outside air, for example, by an aseptic connection device, a tube sealer, or the like. The inlet tube 12 has a first end 12a on the upstream side and a second end 12b on the downstream side. The first end 12a is welded and sealed in the initial state. The second end 12b is connected to an upper end of the collection bag 14.

The collection bag 14 has two flexible sheets 22 overlapping each other in the thickness direction. The two flexible sheets 22 are welded and joined to each other at a plurality of welding portions. Each of the flexible sheets 22 includes a first region 22a for forming one of accommodation chambers (a first accommodation chamber 26), a second region 22b for forming the other accommodation chamber (a second accommodation chamber 28), and a connection region 22c connecting the first region 22a and the second region 22b. The first region 22a has a rectangular shape having the same dimension as the second region 22b in a plan view.

The connection region 22c extends in the width direction and is integrally connected to the first region 22a and the second region 22b. Each of the flexible sheets 22 has a pair of cutouts 22d above and below the connection region 22c, the cutouts 22d being obtained by cutting out the flexible sheet 22 in a rectangular shape. The connection region 22c is formed between the upper and lower cutouts 22d. Although not particularly limited, the connection region 22c is located at the center in the vertical direction of the first region 22a and the second region 22b.

Each of the flexible sheets 22 further includes an upper connection region 22e that connects the upper part of the first region 22a and the upper part of the second region 22b, and a lower connection region 22f that connects the lower part of the first region 22a and the lower part of the second region 22b. The collection bag 14 is reinforced by the upper connection region 22e and the lower connection region 22f.

The collection bag 14 includes the first accommodation chamber 26, the second accommodation chamber 28, and a connection flow path 30 which are defined by a welding portion. The first accommodation chamber 26 is formed in the first regions 22a of the flexible sheets 22. The first accommodation chamber 26 accommodates the platelet product to be introduced into the first culture bottle 90. The first accommodation chamber 26 bulges in the thickness direction of the flexible sheets 22 in the initial state (state at the time of being provided as a product). In the first accommodation chamber 26, one of the flexible sheets 22 and the other flexible sheet 22 are separated from each other in the thickness direction. The first accommodation chamber 26 has a volume for accommodating a prescribed amount (for example, from about 8 mL to about 10 mL) of the platelet product.

The collection bag 14 has an inlet port 32 and a first outlet port 34 which are connected to the first accommodation chamber 26. The inlet port 32 is connected to an upper end of the first accommodation chamber 26 of the collection bag 14. The inlet port 32 is connected to the second end 12b of the inlet tube 12, and allows the inlet tube 12 and the first accommodation chamber 26 to communicate with each other. The first outlet port 34 is connected to a lower end of the first accommodation chamber 26. The first outlet port 34 is connected to the first sealing member 16. The first adapter 21a is connected to the first outlet port 34 via the first sealing member 16.

The second accommodation chamber 28 is formed in the second regions 22b of the flexible sheets 22. The second accommodation chamber 28 accommodates the platelet product to be introduced into the second culture bottle 92. The second accommodation chamber 28 has the same shape as the first accommodation chamber 26 and has a volume for accommodating a prescribed amount (for example, from about 8 mL to about 10 mL) of the platelet product equal to that of the first accommodation chamber 26.

The collection bag 14 has a second outlet port 36 and an exhaust port 38 which are connected to the second accommodation chamber 28. The second outlet port 36 is connected to a lower end of the second accommodation chamber 28 of the collection bag 14. The second outlet port 36 is connected to the second sealing member 18. The second adapter 21b is connected to the second outlet port 36 via the second sealing member 18. The exhaust port 38 is connected to an upper end of the second accommodation chamber 28. The exhaust valve 20 is connected to the exhaust port 38.

The connection flow path 30 is a flow path that connects the first accommodation chamber 26 and the second accommodation chamber 28. An upstream end 30a of the connection flow path 30 is connected to an upper part of the first accommodation chamber 26. The connection flow path 30 extends obliquely upward from the end 30a, is curved at a top portion 30b, and extends downward. The connection flow path 30 extends downward to the center in the vertical direction of the collection bag 14 on the downstream side of the top portion 30b, and is bent in the width direction at a bent portion 30c. The connection flow path 30 has an intermediate portion 30d extending in the width direction so as to be away from the first accommodation chamber 26 on the downstream side of the bent portion 30c.

A middle portion of the connection flow path 30 in a flow path direction is located at the center of the intermediate portion 30d in the width direction. The intermediate portion 30d extends along the connection regions 22c of the flexible sheets 22 and extends to the second regions 22b. The connection flow path 30 has a bent portion 30e on the downstream side of the intermediate portion 30d. The connection flow path 30 is bent downward at the bent portion 30e. The connection flow path 30 changes the orientation at a folded portion 30f on the downstream side of the bent portion 30e, and is connected to a lower part of the second accommodation chamber 28 at a downstream end 30g located on the downstream side of the folded portion 30f.

The connection flow path 30 bulges in the thickness direction in the initial state. In addition, the intermediate portion 30d of the connection flow path 30 forms a tubular portion 31 having a tubular shape in which a portion passing through the connection region 22c is elongated in the width direction. The tubular portion 31 is sealed with a narrow welding portion at an upper end and a lower end.

The collection bag 14 has four corners 14a arranged to surround the first accommodation chamber 26 and the second accommodation chamber 28. Retaining holes 14h are formed in the four corners 14a.

The first sealing member 16 seals the first outlet port 34 in the initial state to prevent passage of fluid between the first accommodation chamber 26 and the first adapter 21a. The first sealing member 16 is a cylindrical member having a breakable plug therein, and when an operation of bending the plug is performed with an internal flow path in the initial state, the plug is broken, and the internal flow path can be opened. The second sealing member 18 is similar to the first sealing member 16, and seals the second outlet port 36 in the initial state to prevent passage of fluid between the second accommodation chamber 28 and the second adapter 21b.

The exhaust valve 20 communicates with the second accommodation chamber 28 and the first accommodation chamber 26 through the exhaust port 38. The exhaust valve 20 internally has a filter that allows passage of gas and prevents passage of liquid. Although not particularly limited, the exhaust valve 20 may have a hydrophilic filter. When the exhaust valve 20 having the hydrophilic filter comes into contact with the platelet product after the first accommodation chamber 26 and the second accommodation chamber 28 are filled with the platelet product, pores of the filter are clogged by liquid, and thereafter, the exhaust valve 20 prevents the passage of gas and liquid. Such an exhaust valve 20 can prevent backflow of air into the second accommodation chamber 28.

The first adapter 21a includes an accommodation cylinder 80 capable of accommodating the neck part of the first culture bottle 90, a tubular needle portion 82 disposed inside the accommodation cylinder 80, and a rubber cover 84 covering the needle portion 82. The accommodation cylinder 80 has a lid 86 that can be opened and closed. The needle portion 82 can puncture a rubber stopper 93 (see Fig. 1) of the first culture bottle 90. When the needle portion 82 of the first adapter 21a penetrates the rubber stopper 93 of the first culture bottle 90 and the first sealing member 16 is opened, the first culture bottle 90 and the first accommodation chamber 26 communicate with each other. When the needle portion 82 punctures the rubber stopper 93 of the first culture bottle 90, the rubber cover 84 is pushed to the proximal side, and the needle portion 82 penetrates the rubber cover 84. When the needle portion 82 is removed from the rubber stopper 93 of the first culture bottle 90, the rubber cover 84 extends by its elastic restoring force to cover the needle portion 82 again.

The second adapter 21b has the same configuration as the first adapter 21a. When the needle portion 82 of the second adapter 21b penetrates a rubber stopper 93 of the second culture bottle 92 and the second sealing member 18 is opened, the second culture bottle 92 and the second accommodation chamber 28 communicate with each other.

Next, the configuration of the collection kit jig 100 illustrated in Fig. 1 will be described.

The collection kit jig 100 includes a kit holding unit 102, a first adapter supporting unit 104, a second adapter supporting unit 106, a first bottle fixing unit 108, a second bottle fixing unit 110, and an elevation mechanism 112.

The kit holding unit 102 is movable in the vertical direction and holds the collection kit 10. The kit holding unit 102 has a base portion 114 fixed to the elevation mechanism 112. The base portion 114 is a plate-shaped member having a thickness in the horizontal direction, and is formed in a quadrangular shape in the present embodiment. Note that the base portion 114 is not particularly limited in shape. The base portion 114 can be vertically displaced by the elevation mechanism 112. A back surface 114b of the base portion 114 is fixed to the elevation mechanism 112.

The kit holding unit 102 includes a plurality of retaining pins 116 protruding from the base portion 114 as a fixing mechanism for fixing the collection bag 14 of the collection kit 10. The plurality of retaining pins 116 protrudes from a front surface 114f of the base portion 114. The plurality of retaining pins 116 can be inserted into the retaining holes 14h formed in the collection bag 14 of the collection kit 10. The plurality of retaining pins 116 is parallel to each other. The plurality of retaining pins 116 includes four retaining pins 116 that retain the four corners 14a of the collection bag 14. Five or more retaining pins 116 may be provided.

A mode of the fixing mechanism is not limited to the plurality of retaining pins 116. As another mode of the fixing mechanism, a clip structure that grips a plurality of places on the peripheral edge of the collection bag 14 may be used.

The kit holding unit 102 includes a clamp unit 118 that closes the connection flow path 30 (see Fig. 2) when the tubular portion 31 of the collection kit 10 is inserted. The clamp unit 118 has a clamp groove 120 having a V shape with its apex facing downward. The clamp unit 118 protrudes from the front surface 114f of the base portion 114. The clamp unit 118 is formed in a U shape (J shape). The clamp unit 118 has a clamping portion 119 extending upward from a lower portion of the U shape. The clamp groove 120 is formed in the clamping portion 119. The clamp unit 118 is disposed at a position surrounded by the four retaining pins 116.

The kit holding unit 102 further includes a tube holding portion 121 capable of holding the inlet tube 12 of the collection kit 10. The tube holding portion 121 has a pair of holding protrusions 121a protruding from the front surface 114f of the base portion 114. The inlet tube 12 is held between the pair of holding protrusions 121a.

The first adapter supporting unit 104 is provided in the kit holding unit 102 and fixes the first adapter 21a. The first adapter supporting unit 104 has a pair of first protrusions 105 protruding from the front surface 114f of the base portion 114 and separated from each other in the horizontal direction. The first adapter supporting unit 104 holds the first adapter 21a between the pair of first protrusions 105. An inner surface of one of the first protrusions 105 and an inner surface of the other first protrusion 105 are holding surfaces for holding the first adapter 21a. The interval between the inner surface of one of the first protrusions 105 and the inner surface of the other first protrusion 105 is preferably set to be slightly smaller than the outer diameter of the first adapter 21a (holding cylinder). Thus, the first adapter 21a can be held with an appropriate fixing force.

The first adapter supporting unit 104 further includes a pressing portion 104a that comes in contact with a shoulder portion (upper surface) of the accommodation cylinder 80 of the first adapter 21a. The pressing portion 104a is provided above the pair of first protrusions 105. When the first adapter 21a is connected to the first culture bottle 90, the pressing portion 104a can prevent the first adapter 21a from being displaced upward relative to the first adapter supporting unit 104.

The second adapter supporting unit 106 is provided in the kit holding unit 102 and fixes the second adapter 21b. The second adapter supporting unit 106 has a pair of second protrusions 107 protruding from the front surface 114f of the base portion 114 and separated from each other in the horizontal direction. The second adapter 21b is held between the pair of second protrusions 107. An inner surface of one of the second protrusions 107 and an inner surface of the other second protrusion 107 are holding surfaces for holding the second adapter 21b. The interval between the inner surface of one of the second protrusions 107 and the inner surface of the other second protrusion 107 is preferably set to be slightly smaller than the outer diameter of the second adapter 21b (accommodation cylinder 80). Thus, the second adapter 21b can be held with an appropriate fixing force.

The second adapter supporting unit 106 further includes a pressing portion 106a that comes in contact with a shoulder portion (upper surface) of the accommodation cylinder 80 of the second adapter 21b. The pressing portion 106a is provided above the pair of second protrusions 107. When the second adapter 21b is connected to the second culture bottle 92, the pressing portion 106a can prevent the second adapter 21b from being displaced upward relative to the second adapter supporting unit 106.

The first adapter supporting unit 104 and the second adapter supporting unit 106 are aligned in the horizontal direction at the same height.

The first bottle fixing unit 108 is disposed below the kit holding unit 102 and fixes the first culture bottle 90. The second bottle fixing unit 110 is disposed below the kit holding unit 102 and fixes the second culture bottle 92. A bottle fixing member 122 is fixed to a base 101 constituting the lower part of the collection kit jig 100. The first bottle fixing unit 108 and the second bottle fixing unit 110 are provided integrally with the bottle fixing member 122.

The first bottle fixing unit 108 is disposed immediately below the first adapter supporting unit 104. The first bottle fixing unit 108 has a U-shaped bottle holding groove 109 that is open at the upper part and the side part. The first culture bottle 90 is held in the bottle holding groove 109. The interval between inner surfaces of the bottle holding groove 109 is preferably set to be slightly smaller than the outer shape of the first culture bottle 90. Thus, the first culture bottle 90 can be held with an appropriate fixing force. The second bottle fixing unit 110 is disposed immediately below the second adapter supporting unit 106. The second bottle fixing unit 110 is configured similarly to the first bottle fixing unit 108, and thus, the detailed description thereof will be omitted.

The elevation mechanism 112 moves the kit holding unit 102 in the vertical direction. The elevation mechanism 112 includes a frame portion 126 fixed to the base 101, a displacement portion 128 supported by the frame portion 126 so as to be movable up and down, and an elevation operation portion 130 attached to the frame portion 126.

The frame portion 126 includes a support column holder 132 fixed to the base 101, a support column 134 supported by the support column holder 132 and protruding upward, and a guide block 136 fixed to the support column 134. The guide block 136 is attached at a right angle to the support column 134 and extends in the horizontal direction. One end (first end 136a) of the guide block 136 is fixed to the support column 134. The other end (second end 136b) of the guide block 136 is fixed to the displacement portion 128. A guide hole 138 penetrating in the vertical direction is formed in the second end 136b of the guide block 136.

The frame portion 126 further includes a subframe 137 disposed between the guide block 136 and the base 101. The subframe 137 extends in the vertical direction. The upper end of the subframe 137 is connected between the first end 136a and the second end 136b of the guide block 136. The lower end of the subframe 137 is supported by the base 101.

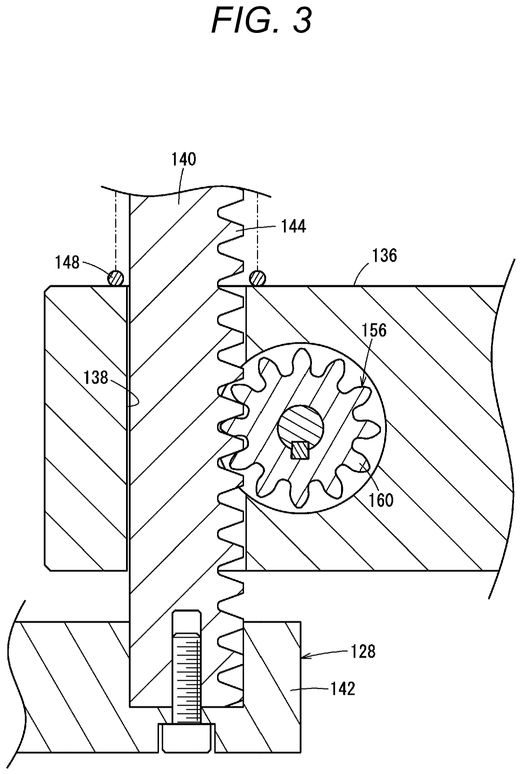

The displacement portion 128 includes an elevation rod 140 inserted into the guide hole 138 of the guide block 136 and a support 142 fixed to a lower end of the elevation rod 140. As illustrated in Fig. 3, a rack 144 is formed on the elevation rod 140 along the extending direction (vertical direction) of the elevation rod 140. As illustrated in Fig. 1, a stationary seat 146 is fixed to an upper end of the elevation rod 140.

A spring 148, which is an example of an elastic member, is disposed between the guide block 136 and the stationary seat 146. The spring 148 is a coil spring. An upper end of the spring 148 is in contact with the stationary seat 146, and a lower end of the spring 148 is in contact with the guide block 136. The elevation rod 140 is inserted into the spring 148. The elastic force of the spring 148 acts to hold the displacement portion 128 in the initial position.

A positioning block 150 is fixed to the stationary seat 146. The positioning block 150 protrudes downward from the lower surface of the stationary seat 146. The lower surface of the positioning block 150 faces the upper surface of the guide block 136. When the displacement portion 128 moves down, the lower end of the positioning block 150 comes in contact with the upper surface of the guide block 136, so that the downward movement of the displacement portion 128 is stopped.

The support 142 is a block-shaped member extending in the horizontal direction. The base portion 114 of the kit holding unit 102 is fixed to the support 142. Two guide rods 152 are inserted into two holes provided at both ends of the support 142. The two guide rods 152 protrude upward from the base 101 in parallel with each other. The guide rods 152 guide the support 142 to move up and down. Note that three or more guide rods 152 may be provided.

The elevation operation portion 130 includes a rotary rod 156 rotatably supported by the guide block 136 and an operation lever 158 fixed to the rotary rod 156. The rotary rod 156 is rotatable about a horizontal axis. As illustrated in Fig. 3, the rotary rod 156 has a gear portion 160 that meshes with the rack 144 of the elevation rod 140. The gear portion 160 is disposed inside the guide block 136. When the gear portion 160 rotates with the rotation of the rotary rod 156, the elevation rod 140 having the rack 144 meshing with the gear portion 160 moves up or down according to the rotation direction of the gear portion 160.

In Fig. 1, the operation lever 158 is a section gripped by a user when the user operates the elevation operation portion 130, and is fixed to the rotary rod 156 at a right angle. The user can rotate the rotary rod 156 by moving the operation lever 158.

The collection kit jig 100 may further include a first electronic balance, a second electronic balance, a first weight indicator, and a second weight indicator. Although none of the first electronic balance, the second electronic balance, the first weight indicator, and the second weight indicator is illustrated, the first electronic balance and the second electronic balance can be provided immediately below the two bottle holding grooves 109 in the bottle fixing member 122, for example. The first weight indicator and the second weight indicator can be provided, for example, on the base 101 or the bottle fixing member 122.

The first electronic balance measures the weight of the object to be sampled transferred into the first culture bottle 90. The first weight indicator indicates the value measured by the first electronic balance. The second electronic balance measures the weight of the object to be sampled transferred into the second culture bottle 92. The second weight indicator indicates the value measured by the second electronic balance. Therefore, the user can quickly grasp the accurate weight of the object to be sampled in each of the first culture bottle 90 and the second culture bottle 92.

The collection kit 10 and the collection kit jig 100 configured as described above are used as follows. In the present embodiment, a collection method for dispensing the platelet product into the two culture bottles 90 and 92 using the collection kit 10 will be described.

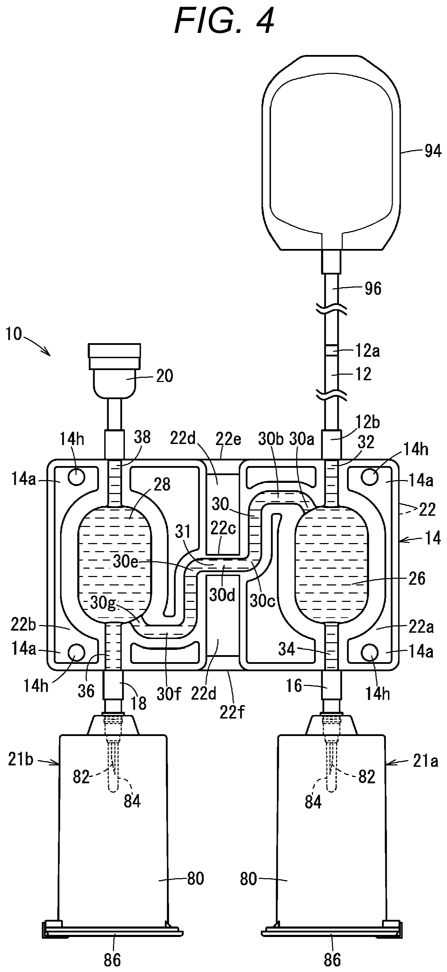

As illustrated in Fig. 4, a platelet bag 94 is connected to the collection kit 10. The platelet bag 94 is a medical bag accommodating the platelet product. The platelet bag 94 has a tube 96 for connection. The tube 96 is connected to the inlet tube 12. The tube 96 is connected to the inlet tube 12 without being exposed to the outside air using an aseptic connection device.

Next, the platelet product is introduced into the collection bag 14. In this step, the platelet bag 94 is placed above the collection bag 14. The first sealing member 16 and the second sealing member 18 of the collection kit 10 are maintained in a closed state.

The platelet product flows out of the platelet bag 94 by gravity and flows into the collection bag 14 through the inlet tube 12. The platelet product flowing into the collection bag 14 flows into the first accommodation chamber 26 through the inlet port 32. The platelet product is stored more and more in the first accommodation chamber 26, and a liquid level of the platelet product in the first accommodation chamber 26 rises. Air inside the first accommodation chamber 26 is pushed out by the platelet product and is discharged from the exhaust valve 20 through the connection flow path 30 and the second accommodation chamber 28.

When the first accommodation chamber 26 is filled with the platelet product, the platelet product flows into the upstream end 30a of the connection flow path 30. Thereafter, the platelet product flows through the connection flow path 30 from upstream to downstream, and flows into the second accommodation chamber 28 from the lower part of the second accommodation chamber 28. As the platelet product continuously flows into the second accommodation chamber 28, a liquid level of the platelet product inside the second accommodation chamber 28 rises. Air in the second accommodation chamber 28 is discharged from the exhaust valve 20. The second accommodation chamber 28 is filled with the platelet product. Furthermore, when the exhaust valve 20 comes into contact with the platelet product, the exhaust valve 20 is closed to prevent the inflow of the platelet product. As a result, the introduction of the platelet product into the collection bag 14 is completed. A prescribed amount (for example, about 8 ml to 10 mL) of the platelet product is accommodated in each of the first accommodation chamber 26 and the second accommodation chamber 28.

Thereafter, the platelet bag 94 is separated from the inlet tube 12 by a tube sealer (high frequency sealer or ultrasonic sealer) or the like. The tube sealer separates the inlet tube 12 from the tube 96 of the platelet bag 94, and at the same time, seals the first end 12a of the inlet tube 12 by welding. The inlet tube 12 and the tube 96 are separated from each other without exposing internal flow paths to the outside air.

The detached platelet bag 94 is stored until a culture test is completed, and then used. In addition, the collection kit 10 filled with the platelet product is carried into a clean bench. The collection kit jig 100 illustrated in Fig. 1 is installed on the clean bench.

In the clean bench, the user attaches the collection kit 10 to the collection kit jig 100 as illustrated in Fig. 1. Specifically, the user inserts the plurality of retaining pins 116 into the plurality of retaining holes 14h provided in the collection bag 14, and fixes the first adapter 21a and the second adapter 21b to the first adapter supporting unit 104 and the second adapter supporting unit 106. Thus, the collection kit 10 is fixed to (held by) the kit holding unit 102.

When the collection kit 10 is attached to the kit holding unit 102, the tubular portion 31 of the collection bag 14 is inserted into the clamp groove 120 of the clamp unit 118. When the tubular portion 31 is inserted into the clamp groove 120, the tubular portion 31 is crushed, so that the connection flow path 30 (see Fig. 2) inside the tubular portion 31 is closed. As a result, the communication between the first accommodation chamber 26 and the second accommodation chamber 28 of the collection bag 14 is blocked, and the movement of the fluid between the first accommodation chamber 26 and the second accommodation chamber 28 is prevented. Note that the tubular portion 31 may be closed by cutting the tubular portion 31 by a tube sealer. Alternatively, the tubular portion 31 may be closed by welding the tubular portion 31 with ultrasonic sealing (or high-frequency sealing). In this case, it is not necessary to provide the clamp unit 118 in the kit holding unit 102.

The user fixes the first culture bottle 90 and the second culture bottle 92 to the first bottle fixing unit 108 and the second bottle fixing unit 110, respectively. The first culture bottle 90 and the second culture bottle 92 may be fixed to the first bottle fixing unit 108 and the second bottle fixing unit 110 before or after the collection kit 10 is fixed to the kit holding unit 102.

When the installation of the collection kit 10, the first culture bottle 90, and the second culture bottle 92 on the collection kit jig 100 is completed as described above, the user then performs an operation for transferring the platelet product from the collection kit 10 to the first culture bottle 90 and the second culture bottle 92.

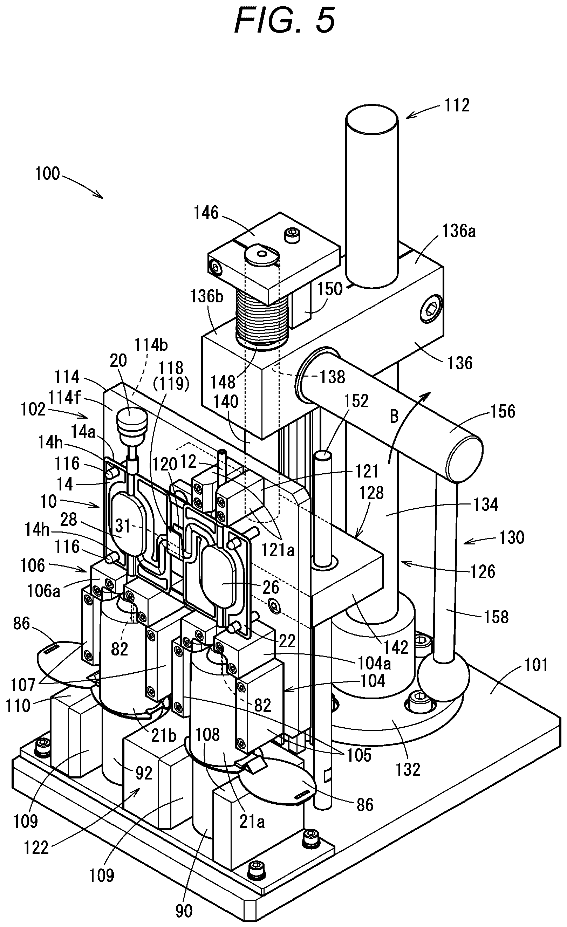

Specifically, the user grips and moves the operation lever 158 in a direction A (see Fig. 1). Thus, the elevation operation portion 130 is rotated as illustrated in Fig. 5. As the rotary rod 156 of the elevation operation portion 130 rotates, the elevation rod 140 having the rack 144 (see Fig. 3) meshing with the gear portion 160 of the rotary rod 156 moves down. As the elevation rod 140 moves down, the base portion 114 fixed to the support 142 moves down, so that the collection kit 10 fixed to the kit holding unit 102 also moves down. When the elevation rod 140 moves down, the spring 148 disposed between the stationary seat 146 and the guide block 136 is compressed in the axial direction. That is, the elevation rod 140 moves down against the elastic force of the spring 148.

As the collection kit 10 moves down, the first culture bottle 90 is connected to the first adapter 21a, and the second culture bottle 92 is connected to the second adapter 21b. The needle portion 82 of the first adapter 21a penetrates the rubber stopper 93 (see Fig. 1) of the first culture bottle 90, and the needle portion 82 of the second adapter 21b penetrates the rubber stopper 93 of the second culture bottle 92.

Thereafter, an operation of opening the first sealing member 16 is performed, the platelet product in the first accommodation chamber 26 is sucked out due to the negative pressure of the first culture bottle 90, and a prescribed amount of the platelet product is introduced into the first culture bottle 90. In addition, an operation of opening the second sealing member 18 is performed, the platelet product in the second accommodation chamber 28 is sucked out due to the negative pressure of the second culture bottle 92, and a prescribed amount of the platelet product is introduced into the second culture bottle 92.

Next, the user operates the elevation operation portion 130 to lift the collection kit 10. Specifically, in Fig. 5, when the elevation operation portion 130 is rotated in a direction B, the kit holding unit 102 moves up. As the kit holding unit 102 moves up, the collection kit 10 held by the kit holding unit 102 also moves up. As the collection kit 10 moves up, the first culture bottle 90 is detached from the first adapter 21a, and the second culture bottle 92 is detached from the second adapter 21b. Through the above steps, the collection of the platelet product using the collection kit 10 is completed.

The collection kit 10 has a structure in which the collection bag 14 is integrally formed using the two flexible sheets 22, and thus can be manufactured at low cost. In addition, the number of times of connection and separation between the tubes can be minimized, whereby the work efficiency is improved. Furthermore, since it is not necessary to perform the work of measuring the platelet product by eye according to the scale line, the work becomes easy and the measurement error can be reduced. In addition, the collection kit 10 can reliably separate the first accommodation chamber 26 and the second accommodation chamber 28 by closing the tubular portion 31 of the collection bag 14 with the clamp unit 118 or the like, thereby being capable of preventing the platelet product from moving between the first accommodation chamber 26 and the second accommodation chamber 28. Therefore, the collection kit 10 can suppress variations in the amount of the platelet product introduced into each of the first culture bottle 90 and the second culture bottle 92.

The collection kit jig 100 according to the present embodiment has the following effects.

The collection kit 10 is held by the kit holding unit 102, the first adapter 21a and the second adapter 21b are fixed to the first adapter supporting unit 104 and the second adapter supporting unit 106, respectively, the first culture bottle 90 and the second culture bottle 92 are fixed to the first bottle fixing unit 108 and the second bottle fixing unit 110, respectively, and the kit holding unit 102 holding the collection kit 10 is lowered by the elevation mechanism 112, whereby the first adapter 21a and the second adapter 21b are connected to the first culture bottle 90 and the second culture bottle 92, respectively. Therefore, the object to be sampled can be easily transferred from the collection kit 10 to the two culture bottles 90 and 92.

Even when the first adapter 21a and the second adapter 21b are provided with the rubber covers 84 covering the needle portions 82 of the first adapter 21a and the second adapter 21b, the first culture bottle 90 and the second culture bottle 92 are not removed from the first adapter 21a and the second adapter 21b by the elastic repulsive force of the rubber stoppers 93.

The kit holding unit 102 includes a base portion 114 fixed to the elevation mechanism 112, and a plurality of retaining pins 116 that can be inserted into retaining holes 14h formed in the collection bag 14 of the collection kit 10 and protrude from the base portion 114. Therefore, the collection kit 10 can be held with a simple configuration.

The collection bag 14 has four corners 14a arranged to surround the first accommodation chamber 26 and the second accommodation chamber 28. The plurality of retaining pins 116 includes four retaining pins 116 that retain the four corners 14a of the collection bag 14. With this configuration, the collection bag 14 can be stably held.

The kit holding unit 102 has a base portion 114 fixed to the elevation mechanism 112. The first adapter supporting unit 104 has a pair of first protrusions 105 protruding from the base portion 114 and separated from each other in the horizontal direction, and holds the first adapter 21a between the pair of first protrusions 105. The second adapter supporting unit 106 has a pair of second protrusions 107 protruding from the base portion 114 and separated from each other in the horizontal direction, and holds the second adapter 21b between the pair of second protrusions 107. With this configuration, the first adapter 21a and the second adapter 21b are satisfactorily positioned, whereby the first adapter 21a and the second adapter 21b can be smoothly connected to the first culture bottle 90 and the second culture bottle 92.

The collection bag 14 has a tubular portion 31 forming the intermediate portion 30d of the connection flow path 30, and the kit holding unit 102 has a clamp unit 118 that closes the connection flow path 30 when the tubular portion 31 is inserted. With this configuration, it is not necessary to attach a separate clamp to the collection kit 10.

The clamp unit 118 has a clamp groove 120 having a V shape with its apex facing downward. The tubular portion 31 of the collection bag 14 is inserted into the V-shaped clamp groove 120 from above the clamp unit 118, by which the connection flow path 30 can be easily and reliably closed.

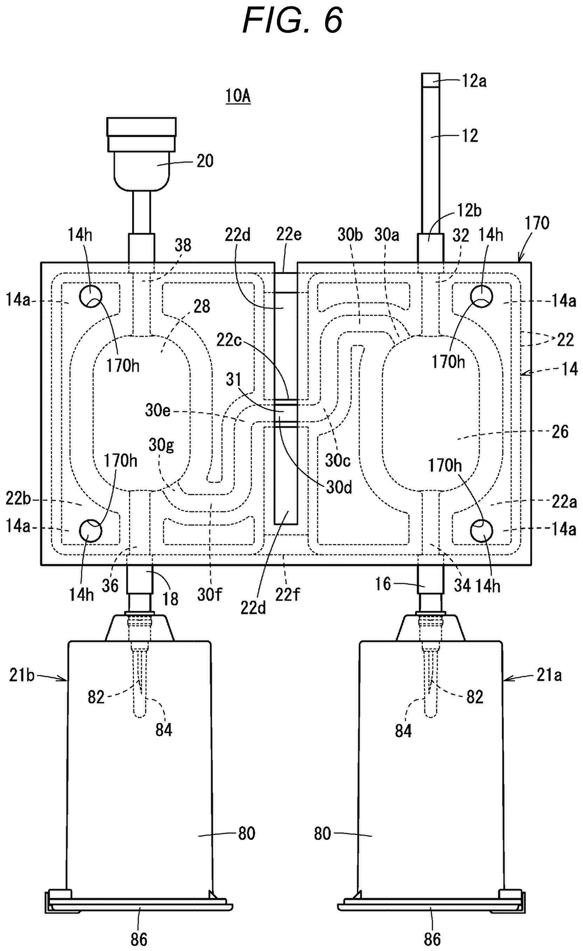

The collection kit jig 100 described above can be used for connecting a collection kit 10A having another configuration illustrated in Fig. 6 to the first culture bottle 90 and the second culture bottle 92. The collection kit 10A is different from the collection kit 10 illustrated in Fig. 2 in further including a holder 170 that holds the collection bag 14.

The holder 170 has a structure of holding the collection bag 14 in the thickness direction. The holder 170 holds the collection bag 14 in the thickness direction to maintain the shape of the collection bag 14 formed from a soft material. The holder 170 keeps a flat shape of the collection bag 14, thereby improving the handleability of the collection kit 10A. In addition, the holder 170 prevents a volume change due to deformation of the first accommodation chamber 26 and the second accommodation chamber 28, and suppresses variations in the amount of the introduced platelet product. The holder 170 has retaining holes 170h at four corners. When the kit holding unit 102 holds the collection kit 10A, the retaining pins 116 are inserted into the retaining holes 170h.

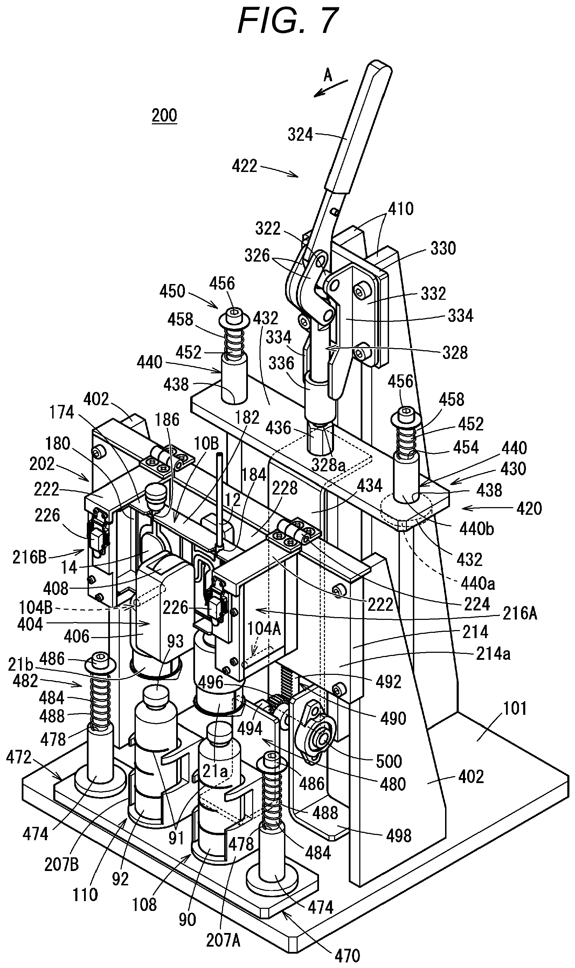

Next, a collection kit jig 200 according to a second embodiment illustrated in Fig. 7 will be described.

The collection kit jig 200 includes a kit holding unit 202, a first adapter supporting unit 104A, a second adapter supporting unit 104B, a first bottle fixing unit 108, a second bottle fixing unit 110, and an elevation mechanism 420.

The kit holding unit 202 is configured to hold a collection kit 10B having a collection bag 14 and a holder 174. The holder 174 has a main wall 180 facing the collection bag 14 and a peripheral wall 182 protruding from an outer periphery of the main wall 180 in a thickness direction of the main wall 180. An inlet tube 12 is held in a fitting hole 184 provided in an upper part of the peripheral wall 182. An exhaust tube 20a (see Fig. 2) of an exhaust valve 20 is held in a positioning hole 186 provided in the upper part of the peripheral wall 182. Although not illustrated in detail, a first sealing member 16 and a second sealing member 18 (see Fig. 2) are respectively held in two engagement holes provided in the lower part of the peripheral wall 182.

Two first support plates 402 are installed upright on the upper surface of a base 101 of the collection kit jig 200. The two first support plates 402 are arranged parallel to each other at the left end and the right end of the base 101. A base portion 214 is positioned and fixed to the upper parts of the front surfaces of the first support plates 402. Therefore, the kit holding unit 202 is positioned and fixed above the first bottle fixing unit 108 and the second bottle fixing unit 110.

The kit holding unit 202 includes a plurality of retainers (retaining tools) that retains the holder 174. Specifically, the plurality of retainers includes a first retainer 216A and a second retainer 216B. The first retainer 216A and the second retainer 216B are provided on the front surface 214a of the base portion 214, protrude forward from the front surface 214a, and extend along the vertical direction.

The first retainer 216A retains the right end part of the holder 174. The first retainer 216A has a lid 222 for opening and closing an upper part of the first retainer 216A. The lid 222 is rotatably connected to the main body of the first retainer 216A via a hinge 224. The first retainer 216A includes a fastener 226 that locks the lid 222 at the closed position.

The second retainer 216B retains the left end part of the holder 174. The second retainer 216B is basically configured similarly to the first retainer 216A. Therefore, in the second retainer 216B, the same names and the same reference numerals are used for the same components as the components of the first retainer 216A.

The front surface 214a of the base portion 214, the first retainer 216A, and the second retainer 216B define an accommodation space 228. The holder 174 is accommodated in the accommodation space 228 in a state of constituting the collection kit 10B by holding the collection bag 14.

The kit holding unit 202 further includes a clamp unit 404. The clamp unit 404 includes a U-shaped stay 406 connected to the base portion 214 and a roller 408 rotatably supported by the U-shaped stay 406. The roller 408 crushes the connection flow path 30 (see Fig. 2) of the collection bag 14. Thus, communication between the first accommodation chamber 26 and the second accommodation chamber 28 (see Fig. 2) is blocked.

The first adapter supporting unit 104A and the second adapter supporting unit 104B are aligned in the horizontal direction at the same height. The first adapter supporting unit 104A is provided in the kit holding unit 202 and fixes the first adapter 21a. The first adapter supporting unit 104A is a pin member protruding from the front surface 214a of the base portion 214. The second adapter supporting unit 104B is provided in the kit holding unit 202 and fixes the second adapter 21b. The second adapter supporting unit 104B is a pin member protruding from the front surface 214a of the base portion 214.

The elevation mechanism 420 moves the first bottle fixing unit 108 and the second bottle fixing unit 110 in the vertical direction. The elevation mechanism 420 includes an elevation operation portion 422, a first displacement portion 430, a first guide portion 450, a second displacement portion 470, a second guide portion 482, and a gear mechanism 490.

Two second support plates 410 are installed upright on the base 101. The elevation operation portion 422 is provided at the upper parts of the front surfaces of the two second support plates 410. The elevation operation portion 422 includes a support shaft 336, an operation lever 324, two V-shaped arms 326, and a connecting rod 328. More specifically, a lever mounting plate 330 is fixed to the upper parts of the front surfaces of the two second support plates 410. A guide bracket 332 is provided on the front surface of the lever mounting plate 330. The guide bracket 332 has two support guide portions 334 extending in the width direction and bent forward. A support shaft 322 is supported by the two support guide portions 334. The support shaft 322 passes through a through hole of the operation lever 324. The operation lever 324 is rotatable about the support shaft 322.

The operation lever 324 has a substantially V-shape. The operation lever 324 is connected to a first movable board 432 of the first displacement portion 430 via the two V-shaped arms 326 and the connecting rod 328. The upper ends of the two V-shaped arms 326 are connected to a bent portion of the V-shaped operation lever 324. The lower ends of the two V-shaped arms 326 are connected to the upper part of the connecting rod 328.

The first displacement portion 430 includes the first movable board 432 and a first drive plate 434. The first movable board 432 and the first drive plate 434 move up and down integrally. The upward and downward movement of the first movable board 432 and the first drive plate 434 is guided by the first guide portion 450.

The first movable board 432 is a plate-shaped member extending along the width direction, and a nut with plate 436 is disposed at substantially the center in the width direction. A male screw portion 328a of the connecting rod 328 is screwed into a female screw of the nut with plate 436. The first movable board 432 has first insertion holes 438 at a left end and a right end, respectively. A cylindrical portion 440b of a first stopper 440 passes through each of the first insertion holes 438. The first stopper 440 is connected to the first movable board 432 in a state where the upper surface of a large-diameter flange 440a of the first stopper 440 is in contact with the lower surface of the first movable board 432.

Two first guide rods 452 constituting the first guide portion 450 are installed upright on the base 101. Each of the two first guide rods 452 passes through a second insertion hole 454 of the first stopper 440. An upper part of each of the two first guide rods 452 is exposed from the second insertion hole 454. A second stopper 456 is provided at the upper end of each of the two first guide rods 452.

The first guide rod 452 passes through a first return spring 458. The lower end of the first return spring 458 is in contact with the upper surface of the first stopper 440. The upper end of the first return spring 458 is in contact with the lower surface of the second stopper 456. The first return spring 458 biases the first movable board 432 downward via the first stopper 440.

The second displacement portion 470 includes a second movable board 472 that is movable up and down. The first bottle fixing unit 108 and the second bottle fixing unit 110 are constituted by a first container holder 207A and a second container holder 207B provided on the upper surface of the second movable board 472, respectively. The first container holder 207A and the second container holder 207B move up and down integrally with the second movable board 472. A scale 91 for weighing is provided on a side surface of the first culture bottle 90 and a side surface of the second culture bottle 92. The bottle fixing member 122 illustrated in Fig. 1 may be used instead of the first container holder 207A and the second container holder 207B.

In Fig. 7, the second movable board 472 has third insertion holes (not illustrated) at the left end and the right end, respectively. The third insertion holes extend in the vertical direction. On the upper surface of the second movable board 472, third stoppers 474 are provided at the left end and the right end, respectively. Each of the third stoppers 474 has a fourth insertion hole 478 that is located above the third insertion hole so as to overlap the third insertion hole. The second drive plate 480 is fixed to the second movable board 472. Therefore, the second movable board 472 moves up and down integrally with the second drive plate 480.

Two second guide rods 484 constituting the second guide portion 482 are installed upright on the base 101. Each of the two second guide rods 484 passes through the third insertion hole and the fourth insertion hole 478. An upper part of each of the two guide rods 362 is exposed from the fourth insertion hole 478. A fourth stopper 486 is provided at the upper end of each of the two guide rods 362.

Each of the second guide rods 484 passes through a second return spring 488. The lower end of the second return spring 488 is in contact with the upper surface of the third stopper 474. The upper end of the second return spring 488 is in contact with the lower surface of the fourth stopper 486. The second return spring 488 biases the second movable board 472 downward via the fourth stopper 486.

The gear mechanism 490 includes a first rack 492, a second rack 494, and a pinion 496 that meshes with the first rack 492 and the second rack 494. The first rack 492 is fixed to the first drive plate 434. The second rack 494 is fixed to the second drive plate 480. The pinion 496 is interposed between the first rack 492 and the second rack 494. The pinion 496 is rotatably supported by an L-shaped stay 498 installed upright on the upper surface of the base 101 via a bearing 500.

The collection kit jig 200 configured as described above is used as follows.

The user sets the collection kit 10B containing an object to be sampled in the kit holding unit 202 of the collection kit jig 200. When setting, the user closes the lid 222, and locks the lid 222 at the closed position by the fastener 226. As the collection kit 10B is set to the kit holding unit 202, the clamp unit 404 presses the connection flow path 30 (see Fig. 2) of the collection bag 14. Thus, communication between the first accommodation chamber 26 and the second accommodation chamber 28 (see Fig. 2) is blocked.

Next, the user performs an operation for transferring the object to be sampled from the collection kit 10B to the first culture bottle 90 and the second culture bottle 92. Specifically, the user grips one end of the operation lever 324 and rotates the operation lever 324 to lower this end in the direction A in Fig. 7. Thus, the connecting rod 328 moves down.

The male screw portion 328a of the connecting rod 328 is connected to the first movable board 432 via the nut with plate 436, and the first drive plate 434 is connected to the first movable board 432. Therefore, as the connecting rod 328 moves down, the first movable board 432 and the first drive plate 434 integrally move down.

When the first movable board 432 and the first drive plate 434 move down, the pinion 496 is pushed downward by the first rack 492 and rotates. As a result, the second rack 494 is pushed upward by the pinion 496. Thus, the second drive plate 480 and the second movable board 472 to which the second drive plate 480 is connected integrally move up.

When the second movable board 472 moves up, the second return spring 488 disposed between the third stopper 474 and the fourth stopper 486 is compressed in the axial direction. That is, the second movable board 472 moves up against the elastic force of the second return spring 488.

The first bottle fixing unit 108 and the second bottle fixing unit 110 integrally move up with the second movable board 472. As a result, the first culture bottle 90 is connected to the first adapter 21a, and the second culture bottle 92 is connected to the second adapter 21b. Thereafter, the first sealing member 16 and the second sealing member 18 (see Fig. 2) are opened by the same operation as in the first embodiment. Accordingly, the object to be sampled is introduced into the first culture bottle 90 and the second culture bottle 92.

Next, the user rotates the operation lever 324 so that one end of the operation lever 324 is raised. Thus, the second return spring 488 is released from the force to compress the second return spring 488 upward. Therefore, the second return spring 488 extends by elasticity. Accordingly, the second movable board 472 is biased downward by the second return spring 488 via the third stopper 474. As a result, the second movable board 472, the first bottle fixing unit 108, and the second bottle fixing unit 110 integrally move down. As the first bottle fixing unit 108 and the second bottle fixing unit 110 move down, the first culture bottle 90 is detached from the first adapter 21a, and the second culture bottle 92 is detached from the second adapter 21b. Through the above steps, the collection (sampling) of the object to be sampled in the first culture bottle 90 and the second culture bottle 92 using the collection kit 10B is completed.

Note that, as the second movable board 472 moves down, the second rack 494 pushes the pinion 496 downward and the pinion 496 pushes the first rack 492 upward, contrary to the above. As a result, the first drive plate 434 and the first movable board 432 move up and return to the original positions.

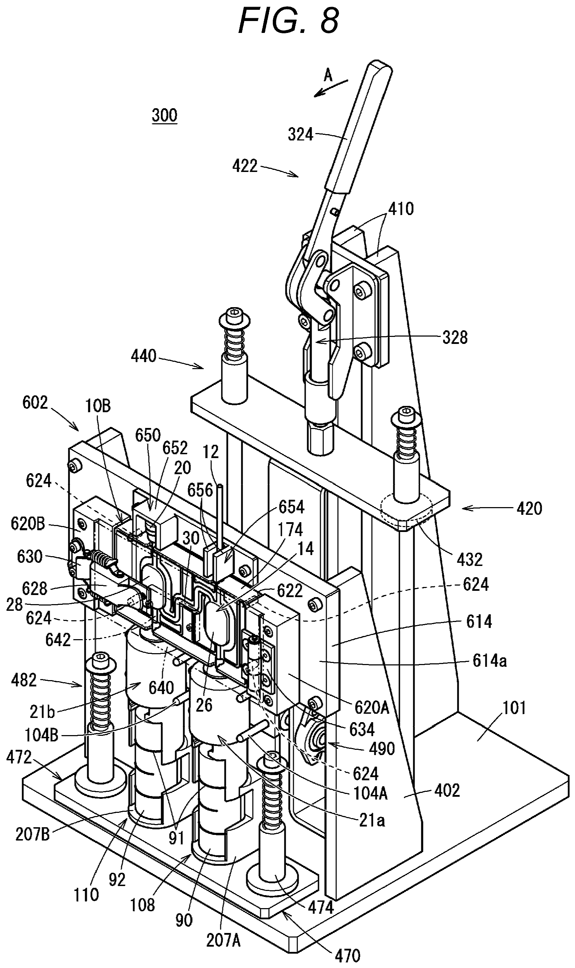

Next, a collection kit jig 300 according to a third embodiment illustrated in Fig. 8 will be described.

The collection kit jig 300 has a configuration obtained by replacing the kit holding unit 202 in the collection kit jig 200 according to the second embodiment illustrated in Fig. 7 with a kit holding unit 602.

In Fig. 8, the kit holding unit 602 includes a base portion 614, a first retainer 620A, a second retainer 620B, a cover 640, a first adapter supporting unit 104A, and a second adapter supporting unit 104B. The first retainer 620A is disposed at the right part of the base portion 614, and the second retainer 620B is disposed at the left part of the base portion 614. A front surface 614a of the base portion 614, the first retainer 620A, and the second retainer 620B define an accommodation space 622 for accommodating a holder 174.

A fastener 628 is provided at the left end of the cover 640. A hinge 634 is connected to the right end of the cover 640. The cover 640 is rotatable via the hinge 634 fixed to the first retainer 620A. The second retainer 620B is provided with a hook 630 for fastening the fastener 628. The cover 640 is preferably transparent or semitransparent. In this case, the user can view the collection bag 14 through the cover 640.

The cover 640 is provided with a clamp unit 642. When the cover 640 is closed, the clamp unit 642 crushes the connection flow path 30 in the collection bag 14, and blocks communication between the first accommodation chamber 26 and the second accommodation chamber 28.

The kit holding unit 602 includes a plurality of retaining pins 624 protruding from the front surface 614a of the base portion 614. The plurality of retaining pins 624 is respectively engaged with four retaining holes provided in the holder 174 of the collection kit 10B. With this configuration, the collection kit 10B can be positioned and fixed to the kit holding unit 602 with a simple structure.

An exhaust valve shielding portion 650 and a tube support 654 are provided on the upper part of the front surface 614a of the base portion 614.

The exhaust valve shielding portion 650 is an enclosure having a roof 652. The roof 652 covers the exhaust valve 20 from above. An upper surface of the exhaust valve 20 and a lower surface of the roof 652 are slightly separated from each other. Accordingly, the exhaust valve 20 is prevented from being closed by the roof 652. The exhaust valve shielding portion 650 suppresses inflow of air into the second accommodation chamber 28 via the exhaust valve 20.

The tube support 654 has a pair of projections 656 protruding forward. The inlet tube 12 of the collection kit 10B is held between the pair of projections 656. Thus, the inlet tube 12 is supported by the tube support 654.

The collection kit jig 300 configured as described above is used as follows.

The user opens the cover 640 and inserts the holder 174 into the accommodation space 622. During insertion, the four retaining pins 624 of the kit holding unit 602 are respectively engaged with the four retaining holes provided in the holder 174. Thus, the collection kit 10B is fixed to (held by) the kit holding unit 602.

Next, the user rotates the cover 640 to close the cover and secures the fastener 628 to the hook 630. Accordingly, the cover 640 is maintained in the closed state.

The operation for transferring the object to be sampled from the collection kit 10B to the first culture bottle 90 and the second culture bottle 92 is similar to that in the second embodiment.

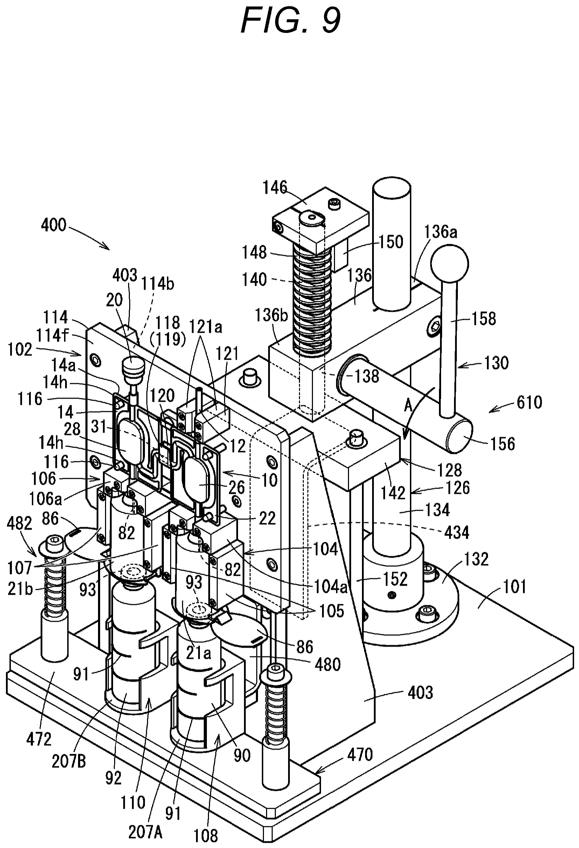

Next, a collection kit jig 400 according to a fourth embodiment illustrated in Fig. 9 will be described.

A kit holding unit 102 of the collection kit jig 400 is configured similarly to the kit holding unit 102 of the collection kit jig 100 illustrated in Fig. 1. In the collection kit jig 400 illustrated in Fig. 9, a base portion 114 of the kit holding unit 102 is fixed to the upper parts of two support plates 403 installed upright on a base 101.

An elevation mechanism 610 of the collection kit jig 400 moves a first bottle fixing unit 108 and a second bottle fixing unit 110 in the vertical direction relative to the kit holding unit 102. The elevation mechanism 610 has a configuration obtained by combining a part of the elevation mechanism 112 illustrated in Fig. 1 and a part of the elevation mechanism 420 illustrated in Fig. 7. In the elevation mechanism 610 illustrated in Fig. 9, the same components as those of the elevation mechanism 112 illustrated in Fig. 1 and the elevation mechanism 420 illustrated in Fig. 7 are denoted by the same reference numerals.

In Fig. 9, the elevation mechanism 610 of the collection kit jig 400 includes a frame portion 126 fixed to the base 101, a displacement portion 128 (hereinafter, referred to as "first displacement portion 128") supported by the frame portion 126 so as to be movable up and down, and an elevation operation portion 130 attached to the frame portion 126. A first drive plate 434 is connected to a support 142 of the displacement portion 128.

The elevation mechanism 610 includes a second displacement portion 470. The second displacement portion 470 includes a second movable board 472 that supports the first bottle fixing unit 108 and the second bottle fixing unit 110, and a second drive plate 480 connected to the second movable board 472. Although not illustrated in Fig. 9, a gear mechanism 490 illustrated in Fig. 7 is disposed between the first drive plate 434 and the second drive plate 480.

When the user rotates the elevation operation portion 130 in the direction A from the state illustrated in Fig. 9, the support 142 of the first displacement portion 128 moves down. The downward movement of the support 142 is converted into the upward movement of the second drive plate 480 by the gear mechanism 490 (see Fig. 7). As the second drive plate 480 moves up, the second movable board 472 moves up, so that the first culture bottle 90 and the second culture bottle 92 respectively supported by the first bottle fixing unit 108 and the second bottle fixing unit 110 move up. As a result, the first culture bottle 90 is connected to the first adapter 21a, and the second culture bottle 92 is connected to the second adapter 21b.

When the user rotates the elevation operation portion 130 in the direction opposite to the direction A after the transfer of the object to be sampled to the first culture bottle 90 and the second culture bottle 92 is completed, the first culture bottle 90 and the second culture bottle 92 move down. Thus, the first culture bottle 90 is detached from the first adapter 21a, and the second culture bottle 92 is detached from the second adapter 21b.

Other possible modifications will be briefly described below.

In the first embodiment illustrated in Fig. 1, the kit holding unit 202 illustrated in Fig. 7 may be provided instead of the kit holding unit 102, and the collection kit 10B may be held in the kit holding unit 202. In the first embodiment illustrated in Fig. 1, the clamp unit 404 illustrated in Fig. 7 may be used instead of the clamp unit 118. In the first embodiment illustrated in Fig. 1, the kit holding unit 602 illustrated in Fig. 8 may be provided instead of the kit holding unit 102, and the collection kit 10B may be held in the kit holding unit 602. In the second embodiment illustrated in Fig. 7, the kit holding unit 102 illustrated in Fig. 1 may be provided instead of the kit holding unit 202. In the third embodiment illustrated in Fig. 8, the kit holding unit 102 illustrated in Fig. 1 may be provided instead of the kit holding unit 602. In the fourth embodiment illustrated in Fig. 9, the elevation mechanism 420 illustrated in Fig. 7 may be used instead of the elevation mechanism 610. In this manner, the components of the first to fourth embodiments may be replaced with each other.

In the first to fourth embodiments, the user operates the operation lever 158, 324 to move the kit holding unit 102, 202, 602 up and down relative to the first bottle fixing unit 108 and the second bottle fixing unit 110. Alternatively, the kit holding unit 102, 202, 602 may be moved up and down relative to the first bottle fixing unit 108 and the second bottle fixing unit 110 by an actuator such as a ball screw or a motor.

In the first to fourth embodiments, the collection kit jig may include a plurality of kit holding units. In this case, when the user operates one operation lever, all the kit holding units are moved up and down relative to the first bottle fixing unit 108 and the second bottle fixing unit 110 at the same time.

The first electronic balance, the second electronic balance, the first weight indicator, and the second weight indicator mentioned in relation to the first embodiment can also be provided in the second to fourth embodiments illustrated in Figs. 7 to 9. In the collection kit jigs 200, 300, and 400 according to the second to fourth embodiments, the first electronic balance and the second electronic balance can be provided on, for example, the second movable board 472.

Specifically, the second movable board 472 includes a main body provided with a first electronic balance and a second electronic balance, and a sliding shelf that can be pulled out forward with respect to the main body. The sliding shelf is positioned immediately below the first container holder 207A and the second container holder 207B, and covers the first electronic balance and the second electronic balance when stored in the main body. On the other hand, when the sliding shelf is pulled out from the main body, the first electronic balance and the second electronic balance are exposed. A through hole is formed at the bottom of each of the first container holder 207A and the second container holder 207B.

In this configuration, when the object to be sampled is transferred from the first accommodation chamber 26 and the second accommodation chamber 28 of the collection bag 14 to the first culture bottle 90 and the second culture bottle 92, respectively, the sliding shelf is pulled forward with respect to the main body. Accordingly, the bottom of the first culture bottle 90 and the bottom of the second culture bottle 92 pass over the through holes and come into contact with the first electronic balance and the second electronic balance, respectively. As a result, the weight of the object to be sampled in the first culture bottle 90 and the weight of the object to be sampled in the second culture bottle 92 are individually measured.

Note that the present invention is not limited to the above disclosure, and various configurations can be adopted without departing from the gist of the present invention.

## Claims

1. A collection kit jig used for connecting a collection kit to a culture bottle,
the collection kit including an inlet tube to which a medical bag containing an object to be sampled is connected, a collection bag connected downstream of the inlet tube, having a first accommodation chamber and a second accommodation chamber, and having a connection flow path connecting the first accommodation chamber and the second accommodation chamber, a first outlet port connected to the first accommodation chamber, a second outlet port connected to the second accommodation chamber, a first adapter connected downstream of the first outlet port and connectable to a first culture bottle, and a second adapter connected downstream of the second outlet port and connectable to a second culture bottle,
the collection kit jig comprising:
a kit holding unit that holds the collection kit;
a first adapter supporting unit that is provided in the kit holding unit and supports the first adapter;
a second adapter supporting unit that is provided in the kit holding unit and supports the second adapter;
a first bottle fixing unit that is disposed below the kit holding unit and fixes the first culture bottle;
a second bottle fixing unit that is disposed below the kit holding unit and fixes the second culture bottle; and
an elevation mechanism that moves the kit holding unit in a vertical direction relative to the first bottle fixing unit and the second bottle fixing unit.

2. The collection kit jig according to claim 1, wherein the kit holding unit includes:
a base portion fixed to the elevation mechanism; and
a plurality of retaining pins that is insertable into retaining holes formed in the collection bag of the collection kit and that protrudes from the base portion.

3. The collection kit jig according to claim 2, wherein
the collection bag has four corners arranged so as to surround the first accommodation chamber and the second accommodation chamber, and
the plurality of retaining pins includes four retaining pins that retain the four corners of the collection bag.

4. The collection kit jig according to claim 1, wherein
the kit holding unit includes a base portion fixed to the elevation mechanism,
the first adapter supporting unit includes a pair of first protrusions protruding from the base portion and separated from each other in a horizontal direction, and holds the first adapter between the pair of first protrusions, and
the second adapter supporting unit has a pair of second protrusions protruding from the base portion and separated from each other in the horizontal direction, and holds the second adapter between the pair of second protrusions.

5. The collection kit jig according to any one of claims 1 to 4, wherein
the collection bag has a tubular portion forming an intermediate portion of the connection flow path, and
the kit holding unit includes a clamp unit that closes the connection flow path when the tubular portion is inserted.

6. The collection kit jig according to claim 5, wherein
the clamp unit has a clamp groove that has a V shape with an apex facing downward.

7. The collection kit jig according to any one of claims 1 to 4, wherein
the elevation mechanism moves the kit holding unit in the vertical direction.

8. The collection kit jig according to any one of claims 1 to 4, wherein
the elevation mechanism moves the first bottle fixing unit and the second bottle fixing unit in the vertical direction.
